# EUROPEAN PATENT APPLICATION

(11) **EP 4 716 249 A1**
(43) Date of publication of application: **25.03.2026**
(21) Application number: 24806872.8
(22) Date of filing: 01.04.2024
(51) Int. Cl.: H04R 1/34

(54) **ULTRASONIC WAVE GENERATING DEVICE**

(30) Priority: 17.05.2023 JP 2023081661
(71) Applicant: Niterra Co., Ltd., Nagoya-shi, Aichi 461-0005 (JP)
(72) Inventor: ITOH Shinsuke, Nagoya-shi, Aichi 461-0005 (JP); YOKOYAMA Kota, Nagoya-shi, Aichi 461-0005 (JP); SUZUKI Satoshi, Nagoya-shi, Aichi 461-0005 (JP); SUZUKI Ryo, Nagoya-shi, Aichi 461-0005 (JP); KASASHIMA Takashi, Nagoya-shi, Aichi 461-0005 (JP)
(74) Representative: Grünecker Patent- und Rechtsanwälte PartG mbB
(86) International application number: PCT/JP2024/013428
(87) International publication number: WO 2024/236918

(57) **Abstract**

An ultrasonic wave generating device (10) includes an ultrasonic wave generating source (11), an ultrasonic wave focusing part (12), and a waveguide (13). The ultrasonic wave focusing part (12) has a first reflection surface (21) and a second reflection surface (22). The first reflection surface (21) reflects ultrasonic waves generated by the ultrasonic wave generating source (11). The second reflection surface (22) reflects the ultrasonic waves reflected by the first reflection surface (21). The first reflection surface (21) and the second reflection surface (22) are arranged such that the ultrasonic waves reflected by the second reflection surface (22) are reflected as plane waves and introduced into the waveguide (13). The ultrasonic wave generating source (11) has a radiation surface (15) that radiates the ultrasonic waves toward the ultrasonic wave focusing part (12). The ultrasonic wave focusing part (12) has a joining surface (20) joined to the radiation surface (15). The joining surface (20) is arranged to be slanted with respect to a direction orthogonal to a traveling direction of the plane waves generated at the second reflection surface (22).

## Description

### Technical Field

The present disclosure relates to an ultrasonic wave generating device.

### Background Art

An ultrasonic wave generating device is disclosed in PTL 1. This ultrasonic wave generating device includes an ultrasonic wave generating source and a focusing part. The focusing part includes a first reflector that reflects at a first reflection surface thereof ultrasonic waves generated by the ultrasonic wave generating source, a second reflector that reflects at a second reflection surface thereof the ultrasonic waves reflected by the first reflection surface, and a waveguide that serves as a transmission path for the ultrasonic waves. The waveguide is disposed such that the ultrasonic waves reflected by the second reflection surface are introduced into the waveguide itself through an introduction part thereof. The focal point of the second reflection surface and the focal point of the first reflection surface are set such that the ultrasonic waves reflected by the second reflection surface are reflected as plane waves.

### Citation List

### Patent Literature

PTL 1: Japanese Unexamined Patent Application Publication No. 2021-90181

### Summary of Invention

### Technical Problem

In the configuration according to PTL 1, the ultrasonic wave generating source needs to generate ultrasonic waves frontward along the axial direction. Accordingly, there is a demand for an ultrasonic wave generating device that offers a higher design flexibility.

The present disclosure is to provide an ultrasonic wave generating device that offers a high design flexibility.

### Solution to Problem

An ultrasonic wave generating device according to the present disclosure is an ultrasonic wave generating device comprising:
an ultrasonic wave generating source that generates ultrasonic waves;
an ultrasonic wave focusing part that focuses the ultrasonic waves generated by the ultrasonic wave generating source; and
a waveguide that transmits the ultrasonic waves focused by the ultrasonic wave focusing part,
wherein the ultrasonic wave focusing part has
a first reflection surface that reflects the ultrasonic waves generated by the ultrasonic wave generating source; and
a second reflection surface that reflects the ultrasonic waves reflected by the first reflection surface,
wherein the first reflection surface and the second reflection surface are arranged such that the ultrasonic waves reflected by the second reflection surface are reflected as plane waves and introduced into the waveguide,
wherein the ultrasonic wave generating source has a radiation surface that radiates the ultrasonic waves toward the ultrasonic wave focusing part,
wherein the ultrasonic wave focusing part has a joining surface joined to the radiation surface, and
wherein the joining surface is arranged to be slanted with respect to a direction orthogonal to a traveling direction of the plane waves generated at the second reflection surface.

### Advantageous Effects of Invention

According to the present disclosure, an ultrasonic wave generating device that offers a high design flexibility can be provided.

### Brief Description of Drawings

[Fig. 1] Fig. 1 is a sectional view of an ultrasonic wave generating device according to a first embodiment.
[Fig. 2] Fig. 2 is a plan view of the ultrasonic wave generating device according to the first embodiment.
[Fig. 3] Fig. 3 is a sectional view of an ultrasonic wave generating device according to a second embodiment.
[Fig. 4] Fig. 4 is a sectional view of an ultrasonic wave generating device according to a third embodiment.
[Fig. 5] Fig. 5 is a sectional view of an ultrasonic wave generating device according to a fourth embodiment.
[Fig. 6] Fig. 6 is a sectional view of an ultrasonic wave generating device according to a fifth embodiment.
[Fig. 7] Fig. 7 is a sectional view of an ultrasonic wave generating device according to a sixth embodiment.
[Fig. 8] Fig. 8 is a sectional view of an ultrasonic wave generating device according to a seventh embodiment.
[Fig. 9] Fig. 9 is a sectional view of an ultrasonic wave generating device according to an eighth embodiment.
[Fig. 10] Fig. 10 is a sectional view of an ultrasonic wave generating device according to a ninth embodiment.
[Fig. 11] Fig. 11 is a sectional view of an ultrasonic wave generating device according to a tenth embodiment.
[Fig. 12] Fig. 12 is a sectional view of an ultrasonic wave generating device according to an eleventh embodiment.
[Fig. 13] Fig. 13 is a diagram illustrating an example based on the ninth embodiment, with a one-side reflection surface and an other-side reflection surface having different curvatures from each other.
[Fig. 14] Fig. 14 is a diagram illustrating another example of a waveguide.

### Description of Embodiments

### [Description of Embodiments of Disclosure]

Embodiments of the present disclosure are listed and exemplified below.
[1] An ultrasonic wave generating device comprising:
   an ultrasonic wave generating source that generates ultrasonic waves;
   an ultrasonic wave focusing part that focuses the ultrasonic waves generated by the ultrasonic wave generating source; and
   a waveguide that transmits the ultrasonic waves focused by the ultrasonic wave focusing part,
   wherein the ultrasonic wave focusing part has
   a first reflection surface that reflects the ultrasonic waves generated by the ultrasonic wave generating source; and
   a second reflection surface that reflects the ultrasonic waves reflected by the first reflection surface,
   wherein the first reflection surface and the second reflection surface are arranged such that the ultrasonic waves reflected by the second reflection surface are reflected as plane waves and introduced into the waveguide,
   wherein the ultrasonic wave generating source has a radiation surface that radiates the ultrasonic waves toward the ultrasonic wave focusing part,
   wherein the ultrasonic wave focusing part has a joining surface joined to the radiation surface, and
   wherein the joining surface is arranged to be slanted with respect to a direction orthogonal to a traveling direction of the plane waves generated at the second reflection surface.
   In the above ultrasonic wave generating device, the joining surface joined to the radiation surface is arranged to be slanted with respect to the direction orthogonal to the traveling direction of the plane waves generated at the second reflection surface. That is, in the above ultrasonic wave generating device, the slant angle of the joining surface can be specified arbitrarily, which offers a high design flexibility.
[2] The ultrasonic wave generating device according to [1],
   wherein the waveguide has a shape extending in a first direction, the first direction being a specific direction among directions orthogonal to the traveling direction, and
   wherein the joining surface is arranged to be slanted with respect to a second direction, the second direction being orthogonal to both the traveling direction and the first direction.
   In the above ultrasonic wave generating device, the ultrasonic waves focused by the ultrasonic wave focusing part can be transmitted over a wide range in the first direction.
[3] The ultrasonic wave generating device according to [1],
   wherein a plurality of said waveguides are arranged at intervals from one another in a first direction, the first direction being a specific direction among directions orthogonal to the traveling direction, and
   wherein the joining surface is arranged to be slanted with respect to a second direction, the second direction being orthogonal to both the traveling direction and the first direction.
   In the above ultrasonic wave generating device, the ultrasonic waves focused by the ultrasonic wave focusing part can be transmitted through each of the plurality of waveguides arranged at intervals in the first direction.
[4] The ultrasonic wave generating device according to [2] or [3],
   wherein the joining surface includes, in the second direction, a one-side joining surface provided on one side relative to the second reflection surface; and an other-side joining surface provided on an other side relative to the second reflection surface, and
   wherein the one-side joining surface and the other-side joining surface have different slant angles from each other with respect to the direction orthogonal to the traveling direction.
   The above ultrasonic wave generating device can have different shapes on the one side and on the other side of the second reflection surface in the second direction.
[5] The ultrasonic wave generating device according to any of [1] to [3],
   wherein a flow path is provided inside the waveguide.
   In the above ultrasonic wave generating device, liquid flows through the flow path, and the ultrasonic waves are transmitted through the liquid, whereby the efficiency of transmission of the ultrasonic waves can be improved.
[6] The ultrasonic wave generating device according to any of [1] to [3],
   wherein the waveguide has a hollow, and
   wherein the hollow is filled with liquid.
   In the above ultrasonic wave generating device, the ultrasonic waves are transmitted through the liquid filling the hollow, whereby the efficiency of transmission of the ultrasonic waves can be improved.
[7] The ultrasonic wave generating device according to any of [1] to [6],
   wherein, when a line passing through a focal point of the first reflection surface and is parallel to the traveling direction is defined as a center line,
   the radiation surface is arranged facing a portion of the first reflection surface, the portion being located on an opposite side across the center line.
   In the above ultrasonic wave generating device, it is easy to increase slant angles of the radiation surface and the joining surface, which makes it easy to reduce the size of the ultrasonic wave generating device in the direction orthogonal to the traveling direction of the plane waves generated at the second reflection surface.
[8] The ultrasonic wave generating device according to any of [1] to [6],
   wherein the joining surface is arranged at a position on a front side in the traveling direction relative to a rear end of the waveguide.

In the above ultrasonic wave generating device, the ultrasonic wave generating source joined to the joining surface can be arranged at a position on the front side in the traveling direction relative to the rear end of the waveguide, which makes it easy to reduce the size of the ultrasonic wave generating device in the traveling direction.

### [Details of Embodiments of Disclosure]

The ultrasonic wave generating device according to the present disclosure is intended for, for example, ultrasonic diagnosis apparatuses, ultrasonic therapy apparatuses, cavitation generating apparatuses, dental scalers, blood coagulation surgery apparatuses (such as ultrasonic scalpels), ultrasonic processing machines, ultrasonic washing machines (such as ultrasonic cleaners), and so forth.

### 1. First Embodiment

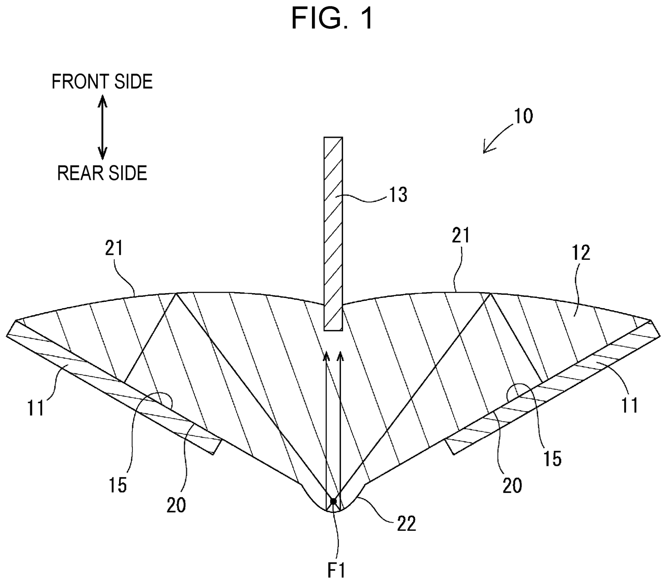

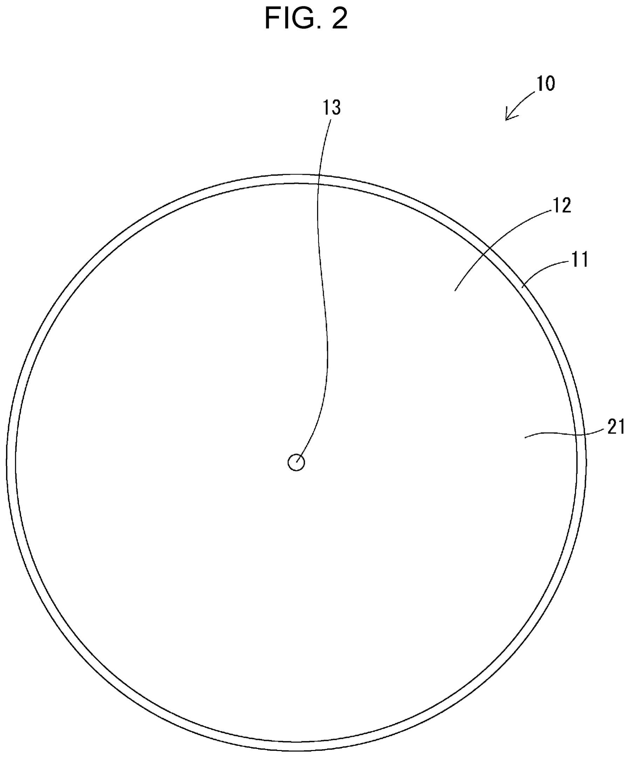

As illustrated in Figs. 1 and 2, an ultrasonic wave generating device 10 includes an ultrasonic wave generating source 11, an ultrasonic wave focusing part 12, and a waveguide 13. The ultrasonic wave generating source 11 generates ultrasonic waves. The ultrasonic wave focusing part 12 focuses the ultrasonic waves generated by the ultrasonic wave generating source 11. The waveguide 13 transmits the ultrasonic waves focused by the ultrasonic wave focusing part 12.

The ultrasonic wave generating source 11, the ultrasonic wave focusing part 12, and the waveguide 13 each have a shape that is obtained when a figure on a plane is rotated by one turn about a straight line given on the plane. That is, the ultrasonic wave generating source 11, the ultrasonic wave focusing part 12, and the waveguide 13 are each a body of revolution.

The ultrasonic wave generating source 11 is, for example, a piezo-electric element. The piezo-electric element includes a piezo-electric body made of piezo ceramic, and electrodes disposed on two sides of the piezo-electric body. The ultrasonic wave generating source 11 has a plate shape. The ultrasonic wave generating source 11 has an annular shape. The ultrasonic wave generating source 11 does not need to have an annular shape, and may be configured by a plurality of piezo-electric elements. The ultrasonic wave generating source 11 is joined to the ultrasonic wave focusing part 12.

When the ultrasonic wave generating source 11 receives an electric signal from the signal transmitter-receiver circuit not illustrated, the ultrasonic wave generating source 11 generates ultrasonic waves. The ultrasonic wave generating source 11 has a radiation surface 15, which radiates ultrasonic waves. The radiation surface 15 has a tapered shape. The ultrasonic waves generated by the ultrasonic wave generating source 11 are plane waves traveling straightly in a direction orthogonal to the radiation surface 15. The ultrasonic wave generating source 11 generates ultrasonic waves at a frequency of, for example, 30 kHz or higher and 10 MHz or lower.

The ultrasonic wave focusing part 12 is made of metal (such as duralumin). The ultrasonic wave focusing part 12 has a circular outline when seen from the front. The ultrasonic wave focusing part 12 has a joining surface 20, which is joined to the ultrasonic wave generating source 11 (more specifically, to the radiation surface 15). The joining surface 20 has a tapered shape. The joining surface 20 receives the ultrasonic waves generated by the ultrasonic wave generating source 11.

The ultrasonic wave focusing part 12 has a first reflection surface 21 and a second reflection surface 22. The second reflection surface 22 is arranged facing the first reflection surface 21. The first reflection surface 21 reflects toward the second reflection surface 22 the ultrasonic waves generated by the ultrasonic wave generating source 11. The second reflection surface 22 reflects toward the rear end of the waveguide 13 the ultrasonic waves reflected by the first reflection surface 21. The first reflection surface 21 and the second reflection surface 22 are arranged such that the ultrasonic waves reflected by the second reflection surface 22 are reflected as plane waves and introduced into the waveguide 13.

The waveguide 13 has a columnar shape (such as a circular cylindrical shape) extending frontward from the front end of the ultrasonic wave focusing part 12. The waveguide 13 extends in a traveling direction of the plane waves generated at the second reflection surface 22. The traveling direction of the plane waves generated at the second reflection surface 22 refers to a direction in which the plane waves generated at the second reflection surface 22 travel within the ultrasonic wave focusing part 12. In the present embodiment, the traveling direction of the plane waves generated at the second reflection surface 22 is a frontward direction. Herein, the term "frontward" refers to a forward side in the traveling direction of the plane waves generated at the second reflection surface 22, and the term "rearward" refers to a backward side in the traveling direction of the plane waves generated at the second reflection surface 22. The direction in which the waveguide 13 extends may be parallel to the traveling direction of the plane waves generated at the second reflection surface 22, may be slightly slanted with respect to the traveling direction of the plane waves generated at the second reflection surface 22, or may be bent.

In the present embodiment, the waveguide 13 is described as a member that is separate from the ultrasonic wave focusing part 12, but the waveguide 13 may be the same member as the ultrasonic wave focusing part 12. The waveguide 13 may preferably be made of a material exhibiting a high propagation capability for ultrasonic waves, and preferable examples of the material include an aluminum alloy, metal glass, and the like. The waveguide 13 may alternatively be made of, for example, a shape-memory alloy composed of titanium and nickel. The waveguide 13 is elastically deformable.

The joining surface 20 of the ultrasonic wave focusing part 12 described above is arranged to be slanted with respect to a direction orthogonal to the traveling direction (that is, the front-rear direction) of the plane waves generated at the second reflection surface 22. The joining surface 20 is slanted frontward while extending away from the waveguide 13 in the direction orthogonal to the front-rear direction. The radiation surface 15 joined to the joining surface 20 is also arranged to be slanted in the same manner as the joining surface 20. The radiation surface 15 is arranged at a position where the ultrasonic waves radiated from the radiation surface 15 do not directly enter the waveguide 13.

The first reflection surface 21 is arranged at a position on the front side relative to the rear end of the radiation surface 15 and arranged at a position on the front side relative to the rear end of the joining surface 20. The first reflection surface 21 has an annular shape. When seen from the front side, the first reflection surface 21 surrounds the outer periphery of the waveguide 13. When seen from the outside of the ultrasonic wave focusing part 12, a portion of the first reflection surface 21 that is between the radially inner end and the radially outer end thereof is shaped as a curved surface that is convex toward the front side (the side opposite to the ultrasonic wave generating source 11). When seen from the inside of the ultrasonic wave focusing part 12, the first reflection surface 21 has a concave shape.

The second reflection surface 22 is arranged on the rear side relative to the first reflection surface 21, arranged on the rear side relative to the radiation surface 15, and arranged on the rear side relative to the joining surface 20. In the direction orthogonal to the front-rear direction, the second reflection surface 22 is arranged on the inner side relative to the outer peripheral edge of the first reflection surface 21, arranged on the inner side relative to the radiation surface 15, and arranged on the inner side relative to the joining surface 20. When seen from the outside of the ultrasonic wave focusing part 12, the second reflection surface 22 is a curved surface (for example, a paraboloid) that is convex toward the rear side (the side opposite to the first reflection surface 21). When seen from the inside of the ultrasonic wave focusing part 12, the second reflection surface 22 has a concave shape. The second reflection surface 22 protrudes rearward.

When the ultrasonic wave generating source 11 receives an electric signal from the signal transmitter-receiver circuit not illustrated, the ultrasonic wave generating source 11 generates ultrasonic waves from the radiation surface 15 thereof as illustrated in Fig. 1. The ultrasonic waves radiated from the radiation surface 15 are reflected by the first reflection surface 21 and are focused toward a focal point F1 of the first reflection surface 21. The focal point F1 of the first reflection surface 21 is the same as that of the second reflection surface 22. Therefore, the ultrasonic waves having passed through the focal point F1 of the first reflection surface 21 are reflected by the second reflection surface 22 and introduced as plane waves into the waveguide 13.

As described above, in the ultrasonic wave generating device 10 according to the first embodiment, the joining surface 20 joined to the radiation surface 15 is arranged to be slanted with respect to the direction orthogonal to the traveling direction of the plane waves generated at the second reflection surface 22. That is, in the ultrasonic wave generating device 10, the slant angle of the joining surface 20 can be specified arbitrarily, which offers a high design flexibility. In this specification, the term "slant angle" refers to a slant angle with respect to the direction orthogonal to the traveling direction (the front-rear direction) of the plane waves generated at the second reflection surface 22. Furthermore, it is easy to reduce the size of the ultrasonic wave generating device 10 in the direction orthogonal to the traveling direction of the plane waves generated at the second reflection surface 22.

### 2. Second Embodiment

A second embodiment describes a configuration in which the radiation surface is arranged facing a portion of the first reflection surface that is located on the opposite side across a center line. The center line refers to a line passing through the focal point of the first reflection surface and being parallel to the traveling direction of the plane waves generated at the second reflection surface.

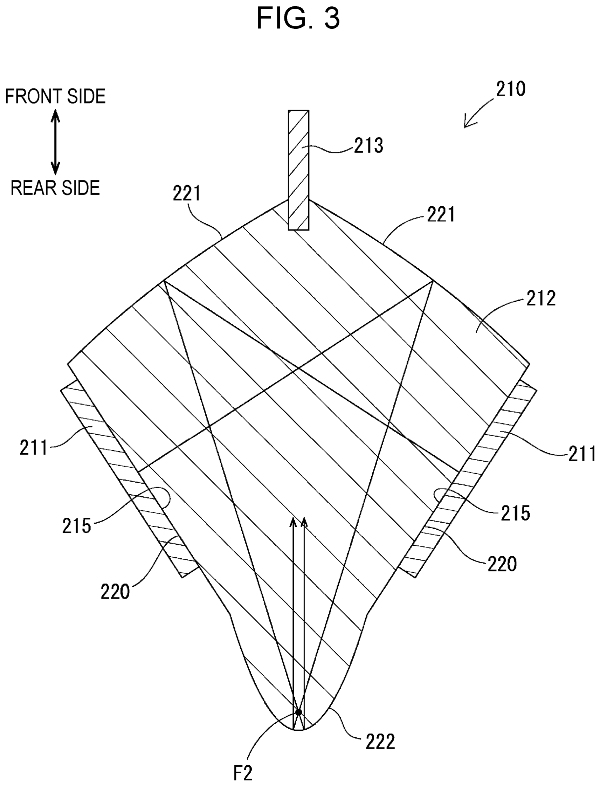

As illustrated in Fig. 3, an ultrasonic wave generating device 210 according to the second embodiment includes an ultrasonic wave generating source 211, an ultrasonic wave focusing part 212, and a waveguide 213. The ultrasonic wave generating source 211, the ultrasonic wave focusing part 212, and the waveguide 213 are each a body of revolution, as with the ultrasonic wave generating source 11, the ultrasonic wave focusing part 12, and the waveguide 13 described in the first embodiment.

The ultrasonic wave generating source 211 is different in shape from the ultrasonic wave generating source 11 described in the first embodiment, but is the same in constituent materials and functions. The ultrasonic wave focusing part 212 is different in shape from the ultrasonic wave focusing part 12 described in the first embodiment, but is the same in constituent materials and functions. The waveguide 213 is different in shape from the waveguide 13 described in the first embodiment, but is the same in constituent materials and functions.

The ultrasonic wave generating source 211 has a radiation surface 215, which radiates ultrasonic waves. The radiation surface 215 has a tapered shape. The ultrasonic wave focusing part 212 has a joining surface 220, which is joined to the ultrasonic wave generating source 211 (more specifically, to the radiation surface 215). The joining surface 220 has a tapered shape. The ultrasonic wave focusing part 212 has a first reflection surface 221 and a second reflection surface 222.

The joining surface 220 of the ultrasonic wave focusing part 212 is arranged to be slanted with respect to a direction orthogonal to the traveling direction of the plane waves generated at the second reflection surface 222. In the present embodiment, the traveling direction of the plane waves generated at the second reflection surface 222 is a frontward direction. The joining surface 220 is slanted frontward while extending away from the waveguide 213 in the direction orthogonal to the front-rear direction. The radiation surface 215 joined to the joining surface 220 is also arranged to be slanted in the same manner as the joining surface 220.

The first reflection surface 221 is arranged at a position on the front side relative to the radiation surface 215 and arranged at a position on the front side relative to the joining surface 220. The first reflection surface 221 has an annular shape. When seen from the front side, the first reflection surface 221 surrounds the outer periphery of the waveguide 213. The inner peripheral edge of the first reflection surface 221 is arranged at a position on the front side relative to the outer peripheral edge of the first reflection surface 221. When seen from the outside of the ultrasonic wave focusing part 212, a portion of the first reflection surface 221 that is between the radially inner end and the radially outer end thereof is shaped as a curved surface that is convex obliquely frontward. When seen from the inside of the ultrasonic wave focusing part 212, the first reflection surface 221 has a concave shape. The first reflection surface 221 and the radiation surface 215 face each other with the center line therebetween. That is, the radiation surface 215 is arranged facing a portion of the first reflection surface 221 that is located on the opposite side across the center line.

The second reflection surface 222 is arranged on the rear side relative to the first reflection surface 221, arranged on the rear side relative to the radiation surface 215, and arranged on the rear side relative to the joining surface 220. In the direction orthogonal to the front-rear direction, the second reflection surface 222 is arranged on the inner side relative to the outer peripheral edge of the first reflection surface 221, arranged on the inner side relative to the radiation surface 215, and arranged on the inner side relative to the joining surface 220. When seen from the outside of the ultrasonic wave focusing part 212, the second reflection surface 222 is a curved surface (for example, a paraboloid) that is convex rearward. When seen from the inside of the ultrasonic wave focusing part 212, the second reflection surface 222 has a concave shape. The second reflection surface 222 protrudes rearward.

When the ultrasonic wave generating source 211 receives an electric signal from a signal transmitter-receiver circuit not illustrated, the ultrasonic wave generating source 211 generates ultrasonic waves from the radiation surface 215 thereof as illustrated in Fig. 3. The ultrasonic waves radiated from the radiation surface 215 are reflected by the portion of the first reflection surface 221 that is located on the opposite side across the center line, and are focused toward a focal point F2 of the first reflection surface 221. The focal point F2 of the first reflection surface 221 is the same as that of the second reflection surface 222. Therefore, the ultrasonic waves having passed through the focal point F2 of the first reflection surface 221 are reflected by the second reflection surface 222 and introduced as plane waves into the waveguide 213.

As described above, in the ultrasonic wave generating device 210 according to the second embodiment, the radiation surface 215 is arranged facing a portion of the first reflection surface 221 that is located on the opposite side across the center line. Therefore, in the ultrasonic wave generating device 210 according to the second embodiment, it is easy to increase slant angles of the first reflection surface 221 and the joining surface 220, which makes it easy to reduce the size of the ultrasonic wave generating device 210 in the direction orthogonal to the front-rear direction.

### 3. Third Embodiment

A third embodiment describes a configuration in which the joining surface is arranged on the front side in the traveling direction relative to the rear end of the waveguide.

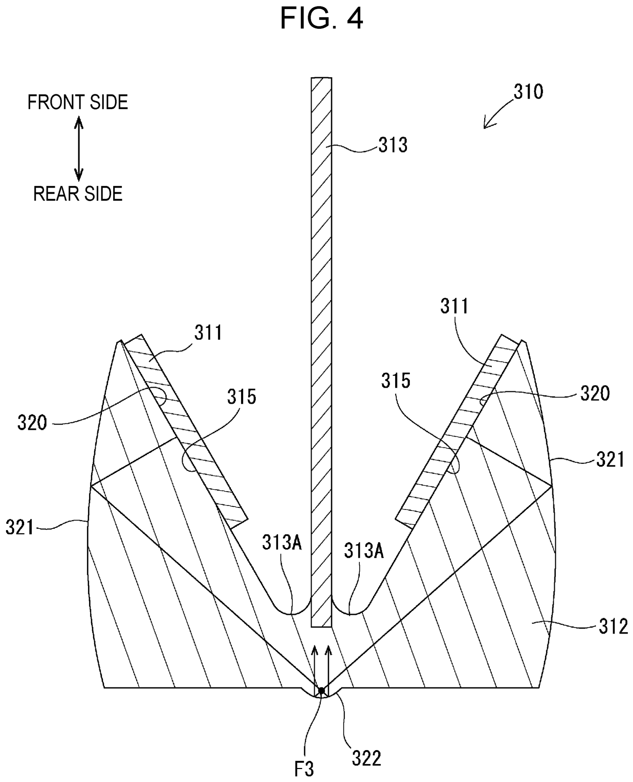

As illustrated in Fig. 4, an ultrasonic wave generating device 310 according to the third embodiment includes an ultrasonic wave generating source 311, an ultrasonic wave focusing part 312, and a waveguide 313. The ultrasonic wave generating source 311, the ultrasonic wave focusing part 312, and the waveguide 313 are each a body of revolution, as with the ultrasonic wave generating source 11, the ultrasonic wave focusing part 12, and the waveguide 13 described in the first embodiment.

The ultrasonic wave generating source 311 is different in shape from the ultrasonic wave generating source 11 described in the first embodiment, but is the same in constituent materials and functions. The ultrasonic wave focusing part 312 is different in shape from the ultrasonic wave focusing part 12 described in the first embodiment, but is the same in constituent materials and functions. The waveguide 313 is different in shape from the waveguide 13 described in the first embodiment, but is the same in constituent materials and functions.

The ultrasonic wave generating source 311 has a radiation surface 315, which radiates ultrasonic waves. The radiation surface 315 has a tapered shape. The ultrasonic wave focusing part 312 has a joining surface 320, which is joined to the ultrasonic wave generating source 311 (more specifically, to the radiation surface 315). The joining surface 320 has a tapered shape. The ultrasonic wave focusing part 312 has a first reflection surface 321 and a second reflection surface 322.

The joining surface 320 of the ultrasonic wave focusing part 312 is arranged to be slanted with respect to a direction orthogonal to the traveling direction of the plane waves generated at the second reflection surface 322. In the present embodiment, the traveling direction of the plane waves generated at the second reflection surface 322 is a frontward direction. The joining surface 320 is arranged on the front side relative to the rear end, 313A, of the waveguide 313. The joining surface 320 faces inward to form a tapered shape with the waveguide 313 as the center axis. The joining surface 320 is slanted frontward while extending away from the waveguide 313 in the direction orthogonal to the front-rear direction. The radiation surface 315 joined to the joining surface 320 is also arranged to be slanted in the same manner as the joining surface 320. The radiation surface 315 is arranged facing the side opposite to the waveguide 313 (specifically, the outer side in the radial direction). In the direction orthogonal to the front-rear direction, the ultrasonic wave generating source 311 is arranged between the waveguide 313 and the joining surface 320.

In the direction orthogonal to the front-rear direction, the first reflection surface 321 is arranged at a position farther away from the waveguide 313 than the radiation surface 315 is, and arranged at a position farther away from the waveguide 313 than the joining surface 320 is. The first reflection surface 321 has an annular shape. When seen from the outside of the ultrasonic wave focusing part 312, the first reflection surface 321 is shaped as a curved surface that is convex toward the side opposite to the waveguide 313 (specifically, the outer side in the radial direction) in the direction orthogonal to the front-rear direction. When seen from the inside of the ultrasonic wave focusing part 312, the first reflection surface 321 has a concave shape.

The second reflection surface 322 is arranged on the rear side relative to the first reflection surface 321, arranged on the rear side relative to the radiation surface 315, and arranged on the rear side relative to the joining surface 320. In the direction orthogonal to the front-rear direction, the second reflection surface 322 is arranged on the inner side relative to the first reflection surface 321, arranged on the inner side relative to the radiation surface 315, and arranged on the inner side relative to the joining surface 320. When seen from the outside of the ultrasonic wave focusing part 312, the second reflection surface 322 is a curved surface (for example, a paraboloid) that is convex rearward. When seen from the inside of the ultrasonic wave focusing part 312, the second reflection surface 322 has a concave shape. The second reflection surface 322 protrudes rearward.

When the ultrasonic wave generating source 311 receives an electric signal from a signal transmitter-receiver circuit not illustrated, the ultrasonic wave generating source 311 generates ultrasonic waves from the radiation surface 315 thereof as illustrated in Fig. 4. The ultrasonic waves radiated from the radiation surface 315 are reflected by the first reflection surface 321 and are focused toward a focal point F3 of the first reflection surface 321. The focal point F3 of the first reflection surface 321 is the same as that of the second reflection surface 322. Therefore, the ultrasonic waves having passed through the focal point F3 of the first reflection surface 321 are reflected by the second reflection surface 322 and introduced as plane waves into the waveguide 313.

As described above, in the ultrasonic wave generating device 310 according to the third embodiment, the joining surface 320 is arranged at a position on the front side relative to the rear end 313A of the waveguide 313. Therefore, in the ultrasonic wave generating device 310 according to the third embodiment, the ultrasonic wave generating source 311 joined to the joining surface 320 can be arranged at a position on the front side relative to the rear end 313A of the waveguide 313, which makes it easy to reduce the size of the ultrasonic wave generating device 310 in the front-rear direction.

### 4. Fourth Embodiment

A fourth embodiment describes a configuration in which the radiation surface is arranged facing a portion of the first reflection surface that is located on the opposite side across the center line, as in the second embodiment. An ultrasonic wave generating device according to the fourth embodiment is different from the ultrasonic wave generating device according to the second embodiment in that the joining surface of the ultrasonic wave focusing part is arranged at a position on the front side relative to the first reflection surface.

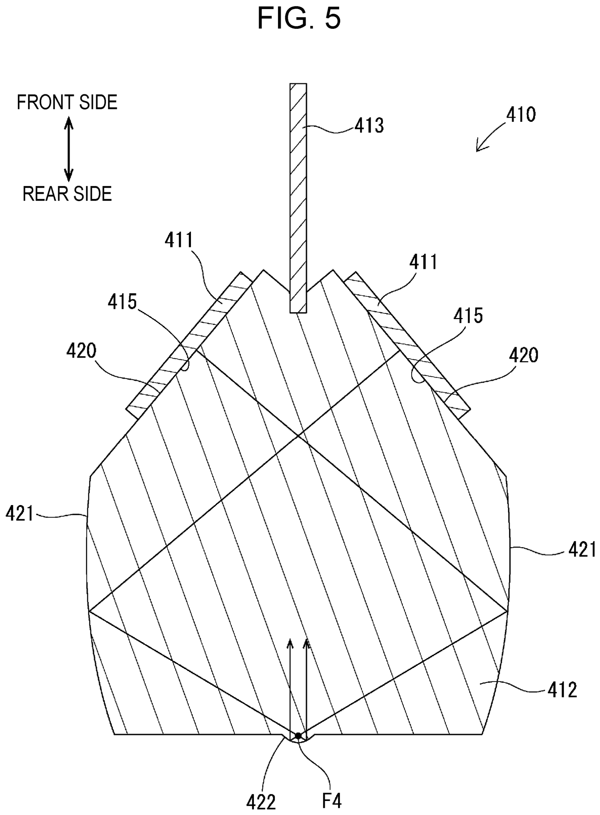

As illustrated in Fig. 5, an ultrasonic wave generating device 410 according to the fourth embodiment includes an ultrasonic wave generating source 411, an ultrasonic wave focusing part 412, and a waveguide 413. The ultrasonic wave generating source 411, the ultrasonic wave focusing part 412, and the waveguide 413 are each a body of revolution, as with the ultrasonic wave generating source 11, the ultrasonic wave focusing part 12, and the waveguide 13 described in the first embodiment.

The ultrasonic wave generating source 411 is different in shape from the ultrasonic wave generating source 11 described in the first embodiment, but is the same in constituent materials and functions. The ultrasonic wave focusing part 412 is different in shape from the ultrasonic wave focusing part 12 described in the first embodiment, but is the same in constituent materials and functions. The waveguide 413 is different in shape from the waveguide 13 described in the first embodiment, but is the same in constituent materials and functions.

The ultrasonic wave generating source 411 has a radiation surface 415, which radiates ultrasonic waves. The radiation surface 415 has a tapered shape. The ultrasonic wave focusing part 412 has a joining surface 420, which is joined to the ultrasonic wave generating source 411 (more specifically, to the radiation surface 415). The joining surface 420 has a tapered shape. The ultrasonic wave focusing part 412 has a first reflection surface 421 and a second reflection surface 422.

The joining surface 420 of the ultrasonic wave focusing part 412 is arranged to be slanted with respect to a direction orthogonal to the traveling direction of the plane waves generated at the second reflection surface 422. In the present embodiment, the traveling direction of the plane waves generated at the second reflection surface 422 is a frontward direction. The joining surface 420 is arranged at a position on the front side relative to the first reflection surface 421. The joining surface 420 is slanted rearward while extending away from the waveguide 413 in the direction orthogonal to the front-rear direction. The radiation surface 415 joined to the joining surface 420 is also arranged to be slanted in the same manner as the joining surface 420.

The first reflection surface 421 is arranged on the rear side relative to the radiation surface 415, arranged on the rear side relative to the joining surface 420, and arranged on the front side relative to the second reflection surface 422. The first reflection surface 421 has an annular shape. When seen from the outside of the ultrasonic wave focusing part 412, the first reflection surface 421 is shaped as a curved surface that is convex toward the side opposite to the waveguide 413 (specifically, the outer side in the radial direction) in the direction orthogonal to the front-rear direction. When seen from the inside of the ultrasonic wave focusing part 412, the first reflection surface 421 has a concave shape. The first reflection surface 421 and the radiation surface 415 face each other with the center line therebetween. That is, the radiation surface 415 is arranged facing a portion of the joining surface 420 that is located on the opposite side across the center line.

The second reflection surface 422 is arranged on the rear side relative to the first reflection surface 421, arranged on the rear side relative to the radiation surface 415, and arranged on the rear side relative to the joining surface 420. In the direction orthogonal to the front-rear direction, the second reflection surface 422 is arranged on the inner side relative to the first reflection surface 421, arranged on the inner side relative to the radiation surface 415, and arranged on the inner side relative to the joining surface 420. When seen from the outside of the ultrasonic wave focusing part 412, the second reflection surface 422 is a curved surface (for example, a paraboloid) that is convex rearward. When seen from the inside of the ultrasonic wave focusing part 412, the second reflection surface 422 has a concave shape. The second reflection surface 422 protrudes rearward.

When the ultrasonic wave generating source 411 receives an electric signal from a signal transmitter-receiver circuit not illustrated, the ultrasonic wave generating source 411 generates ultrasonic waves from the radiation surface 415 thereof as illustrated in Fig. 5. The ultrasonic waves radiated from the radiation surface 415 are reflected by the portion of the first reflection surface 421 that is located on the opposite side across the center line, and are focused toward a focal point F4 of the first reflection surface 421. The focal point F4 of the first reflection surface 421 is the same as that of the second reflection surface 422. Therefore, the ultrasonic waves having passed through the focal point F4 of the first reflection surface 421 are reflected by the second reflection surface 422 and introduced as plane waves into the waveguide 413.

As described above, in the ultrasonic wave generating device 410 according to the fourth embodiment, the radiation surface 415 is arranged facing a portion of the joining surface 420 that is located on the opposite side across the center line. Therefore, in the ultrasonic wave generating device 410 according to the fourth embodiment, it is easy to increase the slant angle of the joining surface 420, which makes it easy to reduce the size of the ultrasonic wave generating device 410 in the direction orthogonal to the front-rear direction.

### 5. Fifth Embodiment

The first to fourth embodiments each have described a configuration in which the second reflection surface is a curved surface that is convex frontward. In contrast, a fifth embodiment describes a configuration in which the second reflection surface is a curved surface that is convex rearward.

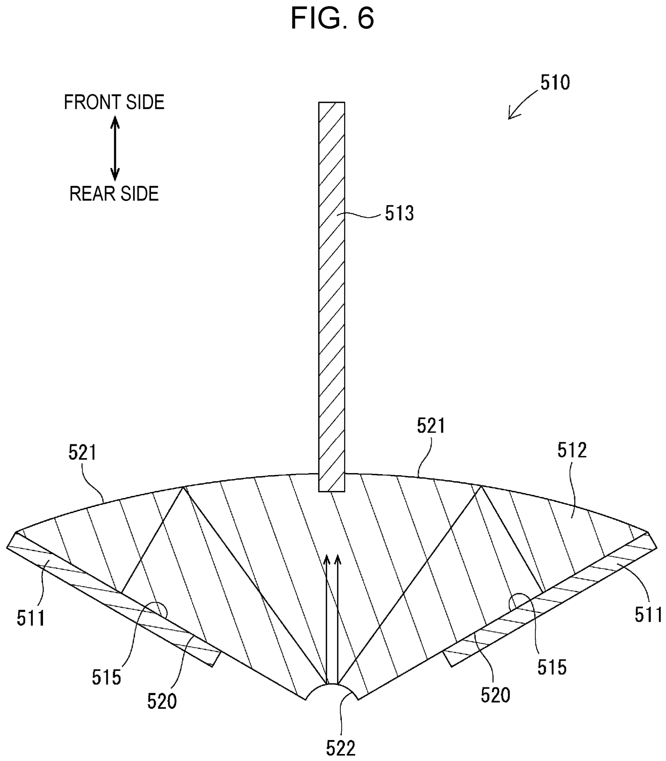

As illustrated in Fig. 6, an ultrasonic wave generating device 510 according to the fifth embodiment includes an ultrasonic wave generating source 511, an ultrasonic wave focusing part 512, and a waveguide 513. The ultrasonic wave generating source 511, the ultrasonic wave focusing part 512, and the waveguide 513 are each a body of revolution, as with the ultrasonic wave generating source 11, the ultrasonic wave focusing part 12, and the waveguide 13 described in the first embodiment.

The ultrasonic wave generating source 511 is different in shape from the ultrasonic wave generating source 11 described in the first embodiment, but is the same in constituent materials and functions. The ultrasonic wave focusing part 512 is different in shape from the ultrasonic wave focusing part 12 described in the first embodiment, but is the same in constituent materials and functions. The waveguide 513 is different in shape from the waveguide 13 described in the first embodiment, but is the same in constituent materials and functions.

The ultrasonic wave generating source 511 has a radiation surface 515, which radiates ultrasonic waves. The radiation surface 515 has a tapered shape. The ultrasonic wave focusing part 512 has a joining surface 520, which is joined to the ultrasonic wave generating source 511 (more specifically, to the radiation surface 515). The joining surface 520 has a tapered shape. The ultrasonic wave focusing part 512 has a first reflection surface 521 and a second reflection surface 522.

The joining surface 520 of the ultrasonic wave focusing part 512 is arranged to be slanted with respect to a direction orthogonal to the traveling direction of the plane waves generated at the second reflection surface 522. In the present embodiment, the traveling direction of the plane waves generated at the second reflection surface 522 is a frontward direction. The joining surface 520 has a tapered shape. The joining surface 520 is slanted frontward while extending away from the waveguide 513 in the direction orthogonal to the front-rear direction. The radiation surface 515 joined to the joining surface 520 is also arranged to be slanted in the same manner as the joining surface 520.

The first reflection surface 521 is arranged at a position on the front side relative to the radiation surface 515 and arranged at a position on the front side relative to the joining surface 520. The first reflection surface 521 has an annular shape. When seen from the front side, the first reflection surface 521 surrounds the outer periphery of the waveguide 513. The inner peripheral edge of the first reflection surface 521 is arranged at a position on the front side relative to the outer peripheral edge of the first reflection surface 521. When seen from the outside of the ultrasonic wave focusing part 12, the first reflection surface 521 is shaped as a curved surface that is convex toward the front side (the side opposite to the ultrasonic wave generating source 511). When seen from the inside of the ultrasonic wave focusing part 512, the first reflection surface 521 has a concave shape.

The second reflection surface 522 is arranged on the rear side relative to the first reflection surface 521. In the direction orthogonal to the front-rear direction, the second reflection surface 522 is arranged on the inner side relative to the outer peripheral edge of the first reflection surface 521, arranged on the inner side relative to the radiation surface 515, and arranged on the inner side relative to the joining surface 520. When seen from the outside of the ultrasonic wave focusing part 512, the second reflection surface 522 is a curved surface (for example, a paraboloid) that is concave frontward. When seen from the inside of the ultrasonic wave focusing part 512, the second reflection surface 522 protrudes frontward.

When the ultrasonic wave generating source 511 receives an electric signal from a signal transmitter-receiver circuit not illustrated, the ultrasonic wave generating source 511 generates ultrasonic waves from the radiation surface 515 thereof as illustrated in Fig. 6. The ultrasonic waves radiated from the radiation surface 515 are reflected by the first reflection surface 521. The ultrasonic waves reflected by the first reflection surface 521 are reflected by the second reflection surface 522 and are introduced into the waveguide 513.

As described above, the second reflection surface 522 may have a shape protruding frontward.

### 6. Sixth Embodiment

The first to fifth embodiments each have described an ultrasonic wave generating device that is a body of revolution. In contrast, a sixth embodiment describes a configuration in which the waveguide has a shape extending in a first direction, which is a specific direction among directions orthogonal to the traveling direction of the plane waves reflected by the second reflection surface.

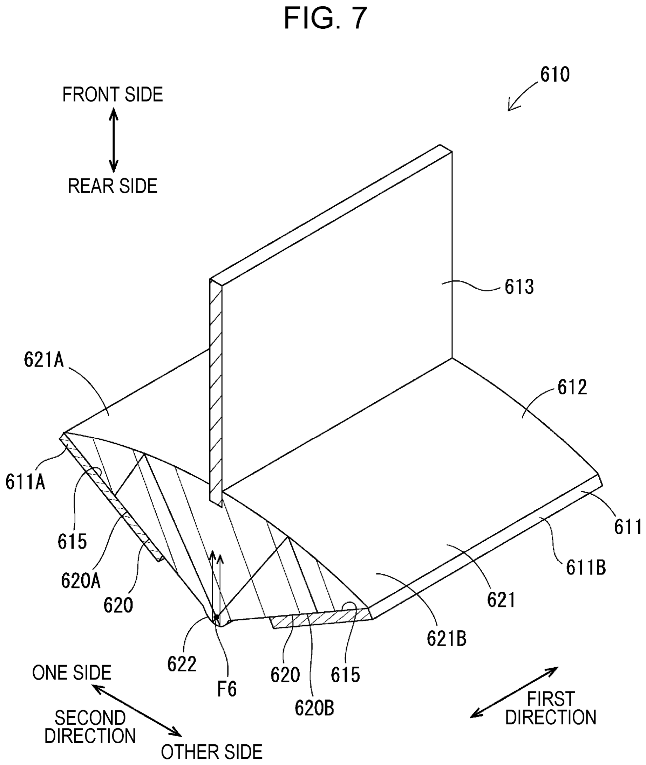

As illustrated in Fig. 7, an ultrasonic wave generating device 610 according to the sixth embodiment includes an ultrasonic wave generating source 611, an ultrasonic wave focusing part 612, and a waveguide 613.

The ultrasonic wave generating source 611 is different in shape from the ultrasonic wave generating source 11 described in the first embodiment, but is the same in constituent materials and functions. The ultrasonic wave focusing part 612 is different in shape from the ultrasonic wave focusing part 12 described in the first embodiment, but is the same in constituent materials and functions. The waveguide 613 is different in shape from the waveguide 13 described in the first embodiment, but is the same in constituent materials and functions.

The ultrasonic wave generating source 611 has a radiation surface 615, which radiates ultrasonic waves. The radiation surface 615 is a flat surface. The ultrasonic wave focusing part 612 has a joining surface 620, which is joined to the ultrasonic wave generating source 611 (more specifically, to the radiation surface 615). The joining surface 620 is a flat surface. The ultrasonic wave focusing part 612 has a first reflection surface 621 and a second reflection surface 622.

When the ultrasonic wave generating source 611 receives an electric signal from a signal transmitter-receiver circuit not illustrated, the ultrasonic wave generating source 611 generates ultrasonic waves from the radiation surface 615 thereof as illustrated in Fig. 7. The ultrasonic waves radiated from the radiation surface 615 are reflected by the first reflection surface 621 and are focused toward a focal point F6 of the first reflection surface 621. The focal point F6 of the first reflection surface 621 is the same as that of the second reflection surface 622. Therefore, the ultrasonic waves having passed through the focal point F6 of the first reflection surface 621 are reflected by the second reflection surface 622 and introduced as plane waves into the waveguide 613.

The joining surface 620 of the ultrasonic wave focusing part 612 is arranged to be slanted with respect to a direction orthogonal to the traveling direction of the plane waves generated at the second reflection surface 622. In the present embodiment, the traveling direction of the plane waves generated at the second reflection surface 622 is a frontward direction. The joining surface 620 is a flat surface extending in the first direction. The joining surface 620 is slanted frontward while extending away from the waveguide 613 in the direction orthogonal to the front-rear direction. The radiation surface 615 joined to the joining surface 620 is also arranged to be slanted in the same manner as the joining surface 620.

The ultrasonic wave generating source 611, the ultrasonic wave focusing part 612, and the waveguide 613 each have a shape extending in the first direction, which is specific among directions orthogonal to the traveling direction of the plane waves generated at the second reflection surface 622.

The joining surface 620 includes, in a second direction, a one-side joining surface 620A, which is provided on one side relative to the second reflection surface 622; and an other-side joining surface 620B, which is provided on an other side relative to the second reflection surface 622. The second direction refers to a direction orthogonal to both the traveling direction of the plane waves generated at the second reflection surface 622 and the first direction. The one-side joining surface 620A and the other-side joining surface 620B have the same slant angle.

The first reflection surface 621 is arranged to be continuous along the first direction. The first reflection surface 621 includes, in the second direction, a one-side reflection surface 621A, which is provided on the one side relative to the waveguide 613; and an other-side reflection surface 621B, which is provided on the other side relative to the waveguide 613. The one-side reflection surface 621A and the other-side reflection surface 621B are each a curved surface (such as a paraboloid) that curves rearward while extending away from the waveguide 613.

The ultrasonic wave generating source 611 includes, in the second direction, a one-side generating source 611A, which is provided on the one side relative to the second reflection surface 622; and an other-side generating source 611B, which is provided on the other side relative to the second reflection surface 622. The one-side generating source 611A and the other-side generating source 611B are each a piezo-electric element. The one-side generating source 611A and the other-side generating source 611B are formed of separate members from each other. Each of the one-side generating source 611A and the other-side generating source 611B may be formed of a single member or may be formed of a plurality of members.

The one-side generating source 611A is arranged facing the one-side reflection surface 621A. The one-side generating source 611A is arranged to be continuous along the first direction. The one-side generating source 611A generates ultrasonic waves over a wide range in the first direction. The one-side reflection surface 621A reflects the ultrasonic waves generated by the one-side generating source 611A. The one-side reflection surface 621A reflects over a wide range in the first direction the ultrasonic waves generated by the one-side generating source 611A.

The other-side generating source 611B is arranged facing the other-side reflection surface 621B. The other-side generating source 611B is arranged to be continuous along the first direction. The other-side generating source 611B generates ultrasonic waves over a wide range in the first direction. The other-side reflection surface 621B reflects the ultrasonic waves generated by the other-side generating source 611B. The other-side reflection surface 621B reflects over a wide range in the first direction the ultrasonic waves generated by the other-side generating source 611B.

The second reflection surface 622 is arranged to be continuous along the first direction. The second reflection surface 622 reflects over a wide range in the first direction the ultrasonic waves reflected by the first reflection surface 621. The ultrasonic waves reflected by the second reflection surface 622 are introduced over a wide range in the first direction into the waveguide 613 extending in the first direction. The waveguide 613 transmits frontward the ultrasonic waves introduced thereinto over a wide range in the first direction.

As described above, in the ultrasonic wave generating device 610 according to the sixth embodiment, the ultrasonic wave focusing part 612 and the waveguide 613 each have a shape extending in the first direction. Therefore, the ultrasonic wave generating device 610 can focus and transmit ultrasonic waves over a wide range in the first direction.

In the ultrasonic wave generating device 610, the one-side generating source 611A corresponding to the one-side reflection surface 621A and the other-side generating source 611B corresponding to the other-side reflection surface 621B are formed of separate members from each other. Therefore, in the ultrasonic wave generating device 610, it is easy to arrange the ultrasonic wave generating sources 611 in an appropriate positional relationship with respect to each of the one-side reflection surface 621A and the other-side reflection surface 621B.

### 7. Seventh Embodiment

The sixth embodiment has described a configuration in which both the ultrasonic wave focusing part and the waveguide each have a shape extending in the first direction. In contrast, a seventh embodiment describes a configuration in which the waveguide has a shape extending in the first direction, whereas the ultrasonic wave focusing part is arranged to be intermittent in the first direction. In the seventh embodiment, elements that are the same as those of the sixth embodiment are denoted by the same reference signs as above, and detailed description of such elements is omitted.

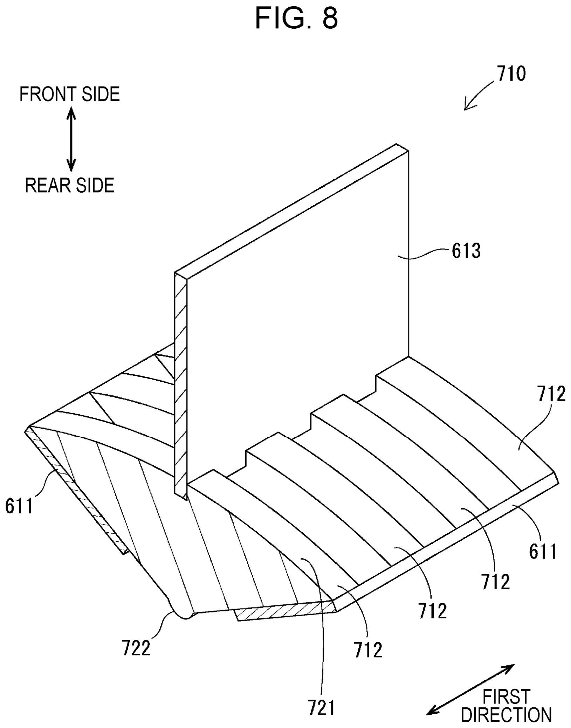

As illustrated in Fig. 8, an ultrasonic wave generating device 710 according to the seventh embodiment includes an ultrasonic wave generating source 611, an ultrasonic wave focusing part 712, and a waveguide 613.

The ultrasonic wave focusing part 712 is constituted such that the ultrasonic wave focusing part 612 described in the sixth embodiment is shortened in the first direction. A plurality of ultrasonic wave focusing parts 712 are provided at intervals from one another in the first direction. The plurality of ultrasonic wave focusing parts 712 are joined to the ultrasonic wave generating source 611 and to the waveguide 613.

Each of the ultrasonic wave focusing parts 712 has a first reflection surface 721 and a second reflection surface 722. Each of the ultrasonic wave focusing parts 712 reflects at the first reflection surface 721 thereof the ultrasonic waves generated by the ultrasonic wave generating source 611. Each of the ultrasonic wave focusing parts 712 causes the ultrasonic waves reflected by the first reflection surface 721 to be reflected by the second reflection surface 722. Each of the ultrasonic wave focusing parts 712 causes the ultrasonic waves reflected by the second reflection surface 722 to be introduced as plane waves into the waveguide 613.

As described above, the ultrasonic wave generating device 710 according to the seventh embodiment can focus and transmit ultrasonic waves over a wide range in the first direction by the plurality of ultrasonic wave focusing parts 712 arranged at intervals from one another in the first direction.

### 8. Eighth Embodiment

The sixth embodiment has described a configuration in which both the ultrasonic wave focusing part and the waveguide each have a shape extending in the first direction. In contrast, an eighth embodiment describes a configuration in which the ultrasonic wave focusing part has a shape extending in the first direction, whereas the waveguide is arranged to be intermittent in the first direction. In the eighth embodiment, elements that are the same as those of the sixth embodiment are denoted by the same reference signs as above, and detailed description of such elements is omitted.

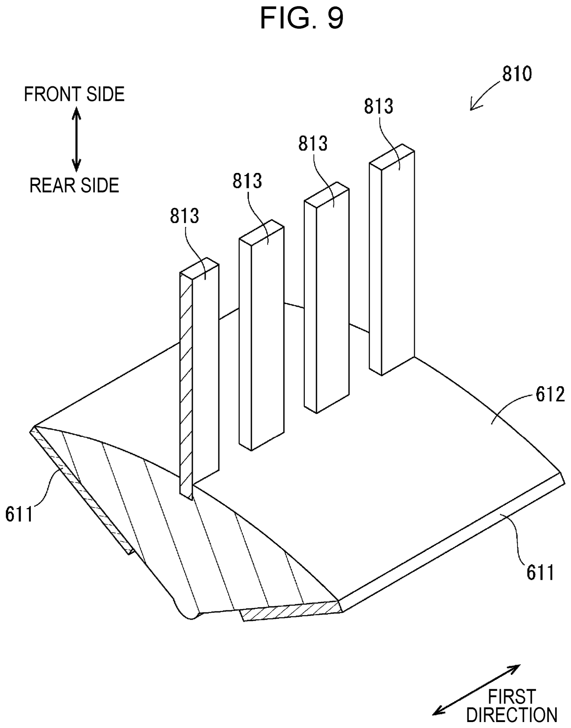

As illustrated in Fig. 9, an ultrasonic wave generating device 810 according to the eighth embodiment includes an ultrasonic wave generating source 611, an ultrasonic wave focusing part 612, and a waveguide 813.

The waveguide 813 has a columnar or needle shape. The waveguide 813 has a shape extending frontward from the front end of the ultrasonic wave focusing part 612. A plurality of waveguides 813 are arranged at intervals from one another in the first direction.

The plane waves generated at the second reflection surface 622 are introduced into each of the waveguides 813 and are transmitted by the waveguides 813.

As described above, in the ultrasonic wave generating device 810 according to the eighth embodiment, the ultrasonic waves focused by the ultrasonic wave focusing part 612 can be transmitted by each of the plurality of waveguides 813 arranged at intervals from one another in the first direction.

### 9. Ninth Embodiment

In the sixth embodiment, the one-side joining surface and the other-side joining surface have the same slant angle. In contrast, a ninth embodiment describes a configuration in which the one-side joining surface and the other-side joining surface have different slant angles from each other.

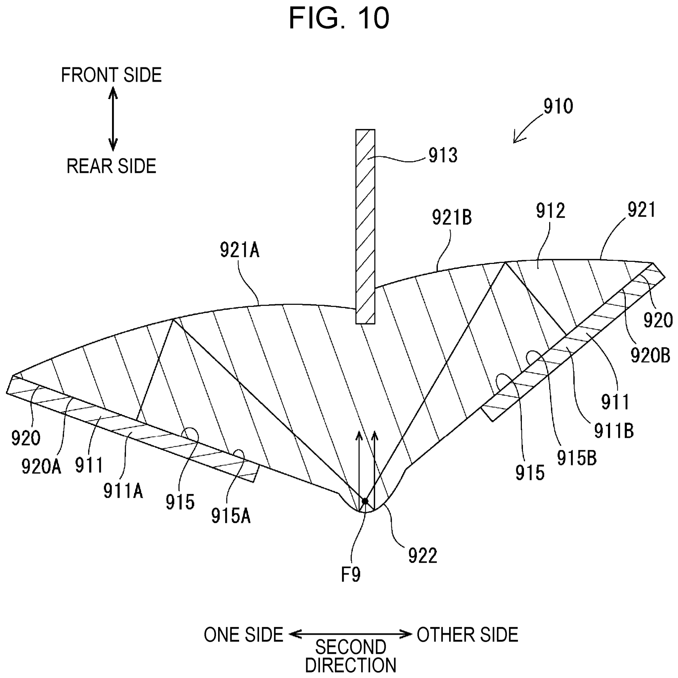

As illustrated in Fig. 10, an ultrasonic wave generating device 910 according to the ninth embodiment includes an ultrasonic wave generating source 911, an ultrasonic wave focusing part 912, and a waveguide 913. The ultrasonic wave generating source 911, the ultrasonic wave focusing part 912, and the waveguide 913 of the ultrasonic wave generating device 910 according to the ninth embodiment each have a shape extending in the first direction, as with those of the ultrasonic wave generating device 610 according to the sixth embodiment.

The ultrasonic wave focusing part 912 has a first reflection surface 921 and a second reflection surface 922. The first reflection surface 921 includes, in the second direction, a one-side reflection surface 921A, which is provided on one side relative to the waveguide 913; and an other-side reflection surface 921B, which is provided on an other side relative to the waveguide 913. The one-side reflection surface 921A and the other-side reflection surface 921B have the same curvature.

The ultrasonic wave generating source 911 includes, in the second direction, a one-side generating source 911A, which is provided on the one side relative to the second reflection surface 922; and an other-side generating source 911B, which is provided on the other side relative to the second reflection surface 922. The ultrasonic wave generating source 911 has a radiation surface 915, which radiates ultrasonic waves. The radiation surface 915 includes, in the second direction, a one-side radiation surface 915A, which is provided on the one side relative to the second reflection surface 922; and an other-side radiation surface 915B, which is provided on the other side relative to the second reflection surface 922. The one-side radiation surface 915A is provided on the one-side generating source 911A. The other-side radiation surface 915B is provided on the other-side generating source 911B.

The ultrasonic wave focusing part 912 has a joining surface 920, which is joined to the radiation surface 915. The joining surface 920 includes, in the second direction, a one-side joining surface 920A, which is provided on the one side relative to the second reflection surface 922; and an other-side joining surface 920B, which is provided on the other side relative to the second reflection surface 922. The one-side joining surface 920A is joined to the one-side radiation surface 915A. The other-side joining surface 920B is joined to the other-side radiation surface 915B. The one-side joining surface 920A and the other-side joining surface 920B have different slant angles from each other.

When the ultrasonic wave generating source 911 receives an electric signal from a signal transmitter-receiver circuit not illustrated, the ultrasonic wave generating source 911 generates ultrasonic waves from the one-side radiation surface 915A and the other-side radiation surface 915B thereof as illustrated in Fig. 10.

The ultrasonic waves radiated from the one-side radiation surface 915A are reflected by the one-side reflection surface 921A and are focused toward a focal point F9 of the one-side reflection surface 921A. The focal point F9 of the one-side reflection surface 921A is the same as that of the second reflection surface 922. Therefore, the ultrasonic waves having passed through the focal point F9 of the one-side reflection surface 921A are reflected by the second reflection surface 922 and introduced as plane waves into the waveguide 913.

The ultrasonic waves radiated from the other-side radiation surface 915B are reflected by the other-side reflection surface 921B and are focused toward the focal point F9 of the other-side reflection surface 921B. The focal point F9 of the other-side reflection surface 921B is the same as that of the second reflection surface 922. Therefore, the ultrasonic waves having passed through the focal point F9 of the other-side reflection surface 921B are reflected by the second reflection surface 922 and introduced as plane waves into the waveguide 913.

### 10. Tenth Embodiment

A tenth embodiment describes a configuration in which a flow path is provided inside the waveguide. The configuration according to the tenth embodiment is also applicable to any of the first to ninth embodiments. In the tenth embodiment, elements that are the same as those of the first embodiment are denoted by the same reference signs as above, and detailed description of such elements is omitted.

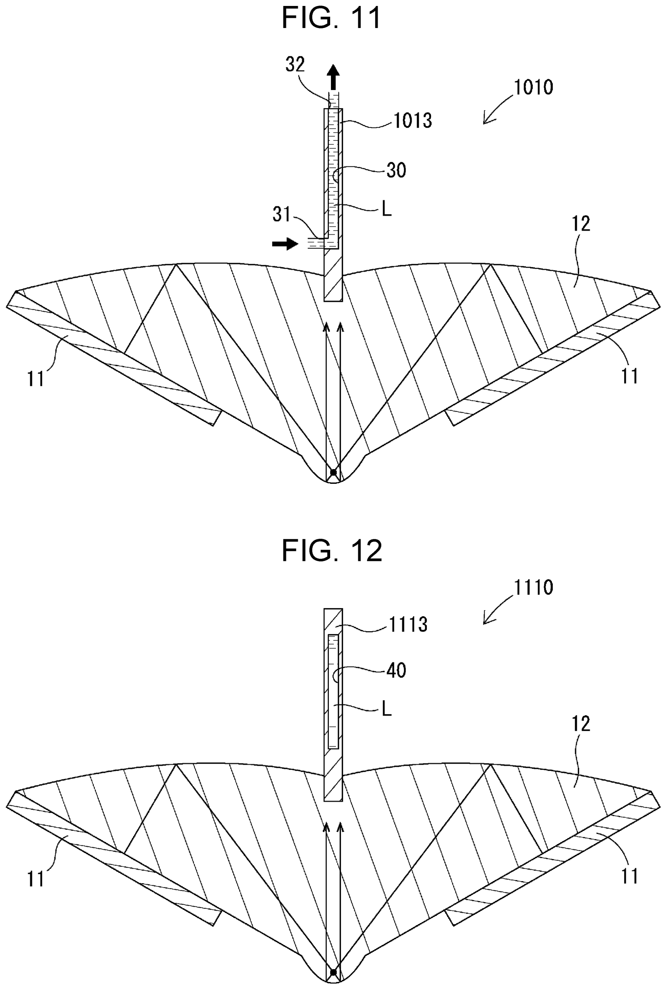

As illustrated in Fig. 11, an ultrasonic wave generating device 1010 according to the tenth embodiment includes an ultrasonic wave generating source 11, an ultrasonic wave focusing part 12, and a waveguide 1013. A flow path 30 is provided inside the waveguide 1013. The waveguide 1013 is different in having the flow path 30 from the waveguide 13 according to the first embodiment, but is the same in other points.

The waveguide 1013 has a columnar shape (such as a circular cylindrical shape) as with the waveguide 13 described in the first embodiment. The flow path 30 has one end 31, which is open at the outer surface of the waveguide 1013. The flow path 30 has an other end 32, which is arranged at the position on the front side relative to the one end 31 and is open at the distal end of the waveguide 1013. Liquid L flows inside the flow path 30. The liquid L flows into the flow path 30 from the one end 31, flows frontward inside the flow path 30, and is discharged from the other end 32. The liquid L is supplied to fill the flow path 30.

In the ultrasonic wave generating device 1010 according to the tenth embodiment, the liquid L flows through the flow path 30, and the ultrasonic waves are transmitted through the liquid L, whereby the efficiency of transmission of the ultrasonic waves can be improved.

### 11. Eleventh Embodiment

An eleventh embodiment describes a configuration in which the waveguide has a hollow filled with liquid. The configuration according to the eleventh embodiment is also applicable to any of the first to tenth embodiments. In the eleventh embodiment, elements that are the same as those of the first embodiment are denoted by the same reference signs as above, and detailed description of such elements is omitted.

As illustrated in Fig. 12, an ultrasonic wave generating device 1110 according to the eleventh embodiment includes an ultrasonic wave generating source 11, an ultrasonic wave focusing part 12, and a waveguide 1113. The waveguide 1113 has a columnar shape (such as a circular cylindrical shape) as with the waveguide 13 described in the first embodiment. The waveguide 1113 has a hollow 40. The waveguide 1113 is different in having the hollow 40 from the waveguide 13 according to the first embodiment, but is the same in other points.

The hollow 40 is hermetically closed. The hollow 40 is filled with liquid L. The liquid L may be gel. The method of feeding the liquid L into the hollow 40 is not limited. For example, the waveguide 1113 may have a hole for exposing the hollow 40, and the liquid L may be fed into the hollow 40 through the hole. The hole is to be closed after the feeding. Alternatively, the waveguide 1113 may have a lid for exposing the hollow 40, and the liquid L may be fed into the hollow 40 with the lid open. The hollow 40 may be wide in the first direction, or a plurality of hollows 40 may be provided at intervals from one another in the first direction.

In the ultrasonic wave generating device 1110 according to the eleventh embodiment, the ultrasonic waves are transmitted through the liquid L filling the hollow 40, whereby the efficiency of transmission of the ultrasonic waves can be improved.

### <Other Embodiments>

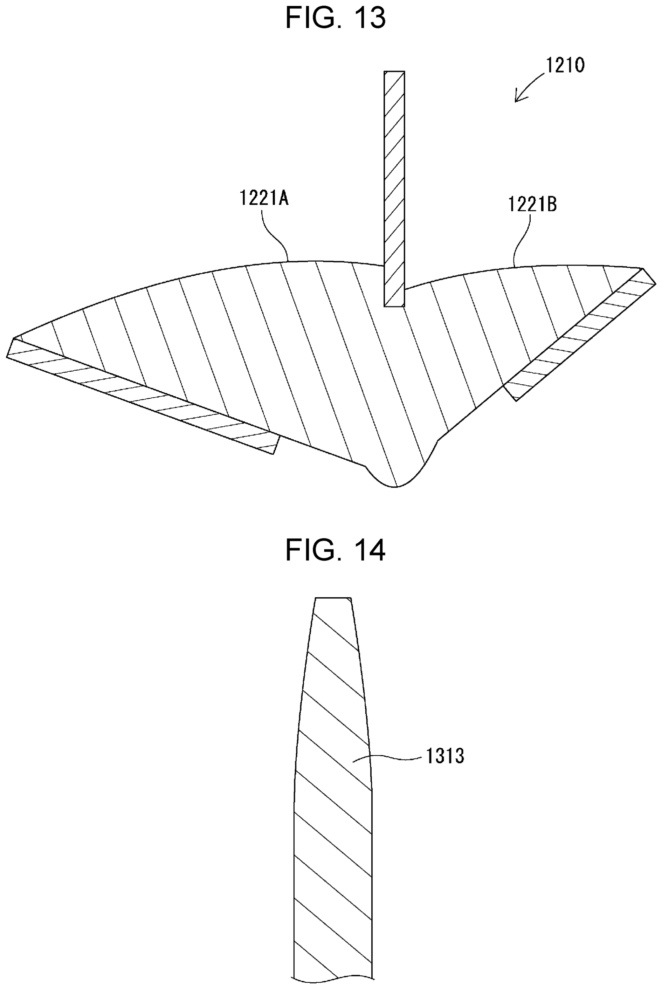

The present invention is not limited to the embodiments described above and illustrated in the drawings. For example, the following embodiments are also within the technical scope of the present invention. Furthermore, various features of the above and following embodiments may be combined in any way, unless the features combined are contradictory to each other.
(1) In the ninth embodiment, the one-side reflection surface and the other-side reflection surface have the same curvature, but may have different curvatures from each other. For example, as in an ultrasonic wave generating device 1210 illustrated in Fig. 13, a one-side reflection surface 1221A and an other-side reflection surface 1221B may have different curvatures from each other.
(2) In the tenth embodiment, the flow path is formed only in the waveguide, but the flow path may extend to the ultrasonic wave focusing part. For example, the flow path may be formed to penetrate the ultrasonic wave focusing part in the front-rear direction.
(3) In the tenth embodiment, the other end of the flow path is formed at the distal end of the waveguide, but may be formed at a halfway position on the peripheral surface of the waveguide.
(4) In each of the above embodiments, the shape of the distal end portion of the waveguide may be different from the shape of a middle portion of the waveguide in the front-rear direction. For example, as with a waveguide 1313 illustrated in Fig. 14, the distal end portion may have a tapered shape.
(5) In each of the above embodiments, the ultrasonic wave generating source is directly joined to the ultrasonic wave focusing part, but may be joined to the ultrasonic wave focusing part with another member therebetween.

It should be understood that the embodiments disclosed herein are only exemplary in all respects and are not limiting. The scope of the present invention is not limited to the embodiments disclosed herein, and is intended to encompass all changes that are made within the scope defined by the claims or within the scope defined by equivalents of the claims.

### Reference Signs List

- 10: ultrasonic wave generating device
- 11: ultrasonic wave generating source
- 12: ultrasonic wave focusing part
- 13: waveguide
- 15: radiation surface
- 20: joining surface
- 21: first reflection surface
- 22: second reflection surface
- 30: flow path
- 31: one end
- 32: other end
- 40: hollow
- 210: ultrasonic wave generating device
- 211: ultrasonic wave generating source
- 212: ultrasonic wave focusing part
- 213: waveguide
- 215: radiation surface
- 220: joining surface
- 221: first reflection surface
- 222: second reflection surface
- 310: ultrasonic wave generating device
- 311: ultrasonic wave generating source
- 312: ultrasonic wave focusing part
- 313: waveguide
- 313A: rear end
- 315: radiation surface
- 320: joining surface
- 321: first reflection surface
- 322: second reflection surface
- 410: ultrasonic wave generating device
- 411: ultrasonic wave generating source
- 412: ultrasonic wave focusing part
- 413: waveguide
- 415: radiation surface
- 420: joining surface
- 421: first reflection surface
- 422: second reflection surface
- 510: ultrasonic wave generating device
- 511: ultrasonic wave generating source
- 512: ultrasonic wave focusing part
- 513: waveguide
- 515: radiation surface
- 520: joining surface
- 521: first reflection surface
- 522: second reflection surface
- 610: ultrasonic wave generating device
- 611: ultrasonic wave generating source
- 611A: one-side generating source
- 611B: other-side generating source
- 612: ultrasonic wave focusing part
- 613: waveguide
- 615: radiation surface
- 620: joining surface
- 620A: one-side joining surface
- 620B: other-side joining surface
- 621: first reflection surface
- 621A: one-side reflection surface
- 621B: other-side reflection surface
- 622: second reflection surface
- 710: ultrasonic wave generating device
- 712: ultrasonic wave focusing part
- 721: first reflection surface
- 722: second reflection surface
- 810: ultrasonic wave generating device
- 813: waveguide
- 910: ultrasonic wave generating device
- 911: ultrasonic wave generating source
- 911A: one-side generating source
- 911B: other-side generating source
- 912: ultrasonic wave focusing part
- 913: waveguide
- 915: radiation surface
- 915A: one-side radiation surface
- 915B: other-side radiation surface
- 920: joining surface
- 920A: one-side joining surface
- 920B: other-side joining surface
- 921: first reflection surface
- 921A: one-side reflection surface
- 921B: other-side reflection surface
- 922: second reflection surface
- 1010: ultrasonic wave generating device
- 1013: waveguide
- 1110: ultrasonic wave generating device
- 1113: waveguide
- 1210: ultrasonic wave generating device
- 1221A: one-side reflection surface
- 1221B: other-side reflection surface
- 1313: waveguide
- F1: focal point
- F2: focal point
- F3: focal point
- F4: focal point
- F6: focal point
- F9: focal point
- L: liquid

## Claims

1. An ultrasonic wave generating device comprising:
an ultrasonic wave generating source that generates ultrasonic waves;
an ultrasonic wave focusing part that focuses the ultrasonic waves generated by the ultrasonic wave generating source; and
a waveguide that transmits the ultrasonic waves focused by the ultrasonic wave focusing part,
wherein the ultrasonic wave focusing part has
a first reflection surface that reflects the ultrasonic waves generated by the ultrasonic wave generating source; and
a second reflection surface that reflects the ultrasonic waves reflected by the first reflection surface,
wherein the first reflection surface and the second reflection surface are arranged such that the ultrasonic waves reflected by the second reflection surface are reflected as plane waves and introduced into the waveguide,
wherein the ultrasonic wave generating source has a radiation surface that radiates the ultrasonic waves toward the ultrasonic wave focusing part,
wherein the ultrasonic wave focusing part has a joining surface joined to the radiation surface, and
wherein the joining surface is arranged to be slanted with respect to a direction orthogonal to a traveling direction of the plane waves generated at the second reflection surface.

2. The ultrasonic wave generating device according to claim 1,
wherein the waveguide has a shape extending in a first direction, the first direction being a specific direction among directions orthogonal to the traveling direction, and
wherein the joining surface is arranged to be slanted with respect to a second direction, the second direction being orthogonal to both the traveling direction and the first direction.

3. The ultrasonic wave generating device according to claim 1,
wherein a plurality of said waveguides are arranged at intervals from one another in a first direction, the first direction being a specific direction among directions orthogonal to the traveling direction, and
wherein the joining surface is slanted with respect to a second direction, the second direction being orthogonal to both the traveling direction and the first direction.

4. The ultrasonic wave generating device according to claim 2 or 3,
wherein the joining surface includes, in the second direction, a one-side joining surface provided on one side relative to the second reflection surface; and an other-side joining surface provided on an other side relative to the second reflection surface, and
wherein the one-side joining surface and the other-side joining surface have different slant angles from each other with respect to a direction orthogonal to the traveling direction.

5. The ultrasonic wave generating device according to any of claims 1 to 3,
wherein a flow path is provided inside the waveguide.

6. The ultrasonic wave generating device according to any of claims 1 to 3,
wherein the waveguide has a hollow, and
wherein the hollow is filled with liquid.
